# EUROPEAN PATENT APPLICATION

(11) **EP 4 417 681 A1**
(43) Date of publication of application: **21.08.2024**
(21) Application number: 22881422.4
(22) Date of filing: 14.10.2022
(51) Int. Cl.: C12M 1/00, C12M 1/33, C12M 1/26, B01D 35/02, B01D 15/34

(54) **EXOSOME EXTRACTION SYSTEM**

(30) Priority: 15.10.2021 KR 20210137564
(71) Applicant: Amogreentech Co., Ltd., Gimpo-si, Gyeonggi-do 10014 (KR)
(72) Inventor: JANG, Seon Ho, Gimpo-si, Gyeonggi-do 10014 (KR); KOO, Song Hee, Gimpo-si, Gyeonggi-do 10014 (KR)
(74) Representative: Treeby, Philip David William
(86) International application number: PCT/KR2022/015650
(87) International publication number: WO 2023/063795

(57) **Abstract**

An exosome extraction system is provided. The exosome extraction system according to one embodiment of the present invention may comprise: a raw material supply unit for supplying a cell fluid containing cells; a cell disruptor which disrupts the cells through physical impact to convert the cell fluid supplied from the raw material supply unit, into a cell lysate including microvesicles, exosomes, and proteins; a filter unit for separating the exosomes from the cell lysate supplied from the cell disruptor; and a recovery unit for recovering the exosomes separated from the filter unit.

## Description

### [Technical Field]

The present invention relates to an exosome extraction system.

### [Background Art]

Exosomes are materials that are produced inside cells and exchange signals between cells.

Because these exosomes contain various types of information such as proteins and DNA of parent cells, exosomes are being developed as biomarkers or therapeutic agents.

These exosomes are naturally secreted from cells during a process of proliferating/culturing cells. Accordingly, a method of centrifuging a medium containing cultured cells is mainly used as a method of obtaining exosomes.

However, although this method can obtain a certain amount of exosomes, the method has problems in that the process is complicated because exosomes are separated through a separate centrifuge after cell culture, and the production yield is low because cell culture and separation are performed separately.

### [Disclosure]

### [Technical Problem]

The present invention is designed to solve the above problems of the related art, and thus it is an object of the present invention to provide an exosome extraction system capable of enhancing production yield while extracting a large amount of exosomes through a single process.

### [Technical Solution]

To solve the above problems, the present invention provides an exosome extraction system, which includes a raw material supply unit configured to supply a liquid-phase cell fluid containing cells; a cell disruptor configured to disrupt the cells through physical impact to convert the cell fluid supplied from the raw material supply unit into a cell lysate including microvesicles, exosomes, and proteins; a filter unit configured to separate the exosomes from the cell lysate supplied from the cell disruptor; and a recovery unit configured to recover the exosomes separated from the filter unit.

In addition, the filter unit may include a primary filter unit connected to the rear end of the cell disruptor to filter or separate proteins from the cell lysate; and a secondary filter unit connected to the rear end of the primary filter unit to filter materials having a larger size than the exosomes from the cell lysate that has passed through the primary filter unit.

Also, the primary filter unit may be a tangential flow filtration (TFF) system.

In addition, the primary filter unit may be a size exclusion chromatography system.

Additionally, the exosome extraction system may include a plurality of primary filter units connected in series with each other between the cell disruptor and the secondary filter unit, and the proteins included in the cell lysate may be filtered multiple times while sequentially passing through the plurality of primary filter units.

Furthermore, the filter unit may include a primary filter unit connected to the rear end of the cell disruptor; and a secondary filter unit connected to the rear end of the primary filter unit to filter the microvesicles from the cell lysate that has passed through the primary filter unit, the primary filter unit may include a plurality of tangential flow filtration (TFF) systems connected in series with each other, and the plurality of TFF systems may include a first TFF system configured to filter materials having a larger size than the microvesicles, the exosomes, and the proteins from the cell lysate, and a second TFF system configured to filter the proteins from the cell lysate that has passed through the first TFF system.

Also, the exosome extraction system may further include a pre-filter unit disposed between the cell disruptor and the filter unit to filter materials having a larger size than the microvesicles, the exosomes, and the proteins from the cell lysate, and the pre-filter unit may include a plate-like filtration member having a predetermined area.

In addition, the filtration member may include a membrane having predetermined pores.

Additionally, the exosome extraction system may further include a filter cake disruption unit configured to disrupt a filter cake attached to a surface of the filtration member.

Furthermore, the filter cake disruption unit may include a buffer solution supply unit that stores a certain amount of a disruption buffer solution supplied to the pre-filter unit, and a supply pump for supplying the disruption buffer solution to the pre-filter unit at a predetermined pressure, the buffer solution supply unit and the supply pump may be connected to the rear end of the pre-filter unit via a supply line, and the disruption buffer solution may be supplied toward the pre-filter unit so that a flow direction of the disruption buffer solution can be opposite to a flow direction of the cell lysate flowing from the cell disruptor to the pre-filter unit.

In this case, the filter cake disrupted from the filtration member through the filter cake disruption unit may be supplied to the raw material supply unit via a bypass line.

Also, the exosome extraction system may further include an additional filter unit connected to the front of the pre-filter unit via the bypass line and an additional recovery unit connected to the rear end of the additional filter unit to recover the exosomes separated from the additional filter unit, the filter cake disrupted from the filtration member through the filter cake disruption unit may be supplied to the additional filter unit along the bypass line, and the additional filter unit may separate the exosomes from the filter cake disrupted from the filtration member.

In addition, the exosome extraction system may further include an additional pre-filter unit connected in parallel with the pre-filter unit via a bypass line connected between the cell disruptor and the pre-filter unit and an additional filter unit and an additional recovery unit sequentially connected to the rear end of the additional pre-filter unit to correspond one-to-one to the additional pre-filter unit, and the cell lysate generated in the cell disruptor may move to any one of the pre-filter unit and the additional pre-filter unit.

Further, the filter unit may include a primary filter unit connected to the rear end of the pre-filter unit to filter the microvesicles from the cell lysate supplied from the pre-filter unit; and a secondary filter unit connected to the rear end of the primary filter unit to separate the proteins and the exosomes from the cell lysate that has passed through the primary filter unit.

### [Advantageous Effects]

According to the present invention, there are advantages in that a large amount of exosomes can be extracted and the production yield can be increased because exosomes can be separated by disrupting a large amount of cells through continuous processing.

### [Description of Drawings]

FIG. 1 is a schematic diagram showing an exosome extraction system according to one embodiment of the present invention.
FIG. 2 is a conceptual diagram showing a filtration method of a TFF system that may be applied to the exosome extraction system according to one embodiment of the present invention.
FIG. 3 is a conceptual diagram showing a separation method of size exclusion chromatography system that can be applied to the exosome extraction system according to one embodiment of the present invention.
FIG. 4 is a diagram showing a modified example of FIG. 1.
FIG. 5 is a diagram showing another modified example of FIG. 1.
FIG. 6 is a schematic diagram showing an exosome extraction system according to another embodiment of the present invention.
FIG. 7 is a schematic diagram showing an exosome extraction system according to still another embodiment of the present invention.
FIG. 8 is a schematic diagram showing a configuration in which the exosome extraction system according to one embodiment of the present invention includes a filter cake disruption unit.
FIG. 9 is a conceptual diagram schematically showing a process in which a filter cake, which is generated on a surface of a filtration member of a pre-filter unit in the exosome extraction system according to one embodiment of the present invention, is disrupted by a disruption buffer solution.
FIG. 10 is a diagram showing a modified example of FIG. 8.
FIG. 11 is a schematic diagram showing an exosome extraction system according to yet another embodiment of the present invention.

### [Modes of the Invention]

Hereinafter, embodiments of the present invention will be described in detail with reference to the accompanying drawings to be easily implemented by those having ordinary skill in the art to which the present invention pertains. However, it should be understood that the present invention may be embodied in various different forms and is not limited to the embodiments set forth herein. In order to clearly describe the present invention, parts not related to the description are omitted, and like parts are denoted by like reference numerals throughout the specification.

An exosome extraction system 100, 100', 200, 300, 400, 500' or 600 according to one embodiment of the present invention may extract exosomes e from a cell lysate generated through a cell disruptor 120, and may extract exosomes e in large quantities through a continuous process.

Here, the cell lysate disrupted through the cell disruptor 120 may be a mixed solution obtained by disrupting a cell fluid containing cells. In this case, the cell lysate may be a mixed solution including undisrupted cells, cell debris, microvesicles, exosomes, and proteins.

Also, the cell fluid and cell lysate may include a medium or a buffer solution.

In this case, the buffer solution may be a solution that serves to protect the cells or exosomes while being mixed with the cells to convert the cells into a liquid-phase cell fluid. Also, the buffer solution may be a solution that does not react with the cells or exosomes. For example, the buffer solution may be the known phosphate-buffered saline (PBS).

In addition, the cells, the cell debris, the microvesicles, the exosomes, and the proteins may have sequentially smaller sizes, and the exosome extraction system 100, 100', 200, or 300 according to one embodiment of the present invention may extract a large amount of high-purity exosomes e by sequentially filtering out materials having different sizes from the cell lysate.

In the present invention, the exosomes e are materials that exchange signals between cells and may have a size of 30 nm to 200 nm or less, and the microvesicles are by-products generated in a process of disrupting cells and may be materials having a larger size than the exosomes. For example, the microvesicles may be an endoplasmic reticulum having a size exceeding 200 nm but 1,000 nm or less. Also, the cell debris may be a by-product generated during a process of disrupting cells and may be a material having a larger size than the microvesicles.

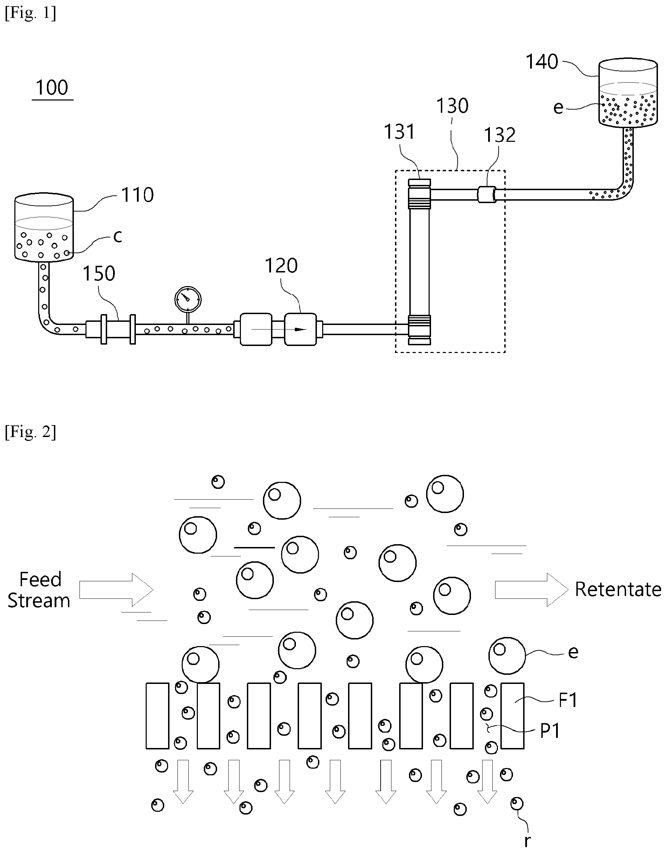

An exosome extraction system 100, 100' or 200 according to one embodiment of the present invention may include a raw material supply unit 110, a cell disruptor 120, a filter unit 130, 130' or 230, and a recovery unit 140, as shown in FIGS. 1, 4, and 5.

The raw material supply unit 110 may serve to supply a cell fluid serving as a raw material for extracting the exosomes e. That is, the raw material supply unit 110 may supply a liquid-phase cell fluid containing cells c to the cell disruptor 120.

For example, the raw material supply unit 110 may be a supply tank that stores a certain amount of the cell fluid, and may be connected to the cell disruptor 120 disposed at the rear end thereof.

Here, the cell fluid may contain the cells c as described above, and may be a mixed solution in which the cells c to be disrupted are mixed with a medium or a buffer solution. For example, the cell fluid may be a mixed solution in which the cells are mixed with a buffer solution.

The cell disruptor 120 may be supplied with a cell fluid from the raw material supply unit 110, and may disrupt the cell fluid into a cell lysate through physical impact.

That is, the cell disruptor 120 may be supplied with a cell fluid from the raw material supply unit 110 using a pump 150, and may disrupt the cell fluid through a physical disruption method using cavitation, shear force, impact, and the like.

In this way, the cell fluid supplied from the raw material supply unit 110 to the cell disruptor 120 may be converted into a cell lysate through the cell disruptor 120.

Here, the cell lysate may be a mixed solution including undisrupted cells, cell debris, microvesicles, exosomes, and proteins, as described above.

That is, the cell fluid supplied to the cell disruptor 120 may be converted into a cell lysate including undisrupted cells, cell debris, microvesicles, exosomes, and proteins.

For example, the cell disruptor 120 may be a high-pressure extruder, but the present invention is not limited thereto. In this case, various known disruptors, such as a high-speed bead mill, an ultrasonicator, a press, and the like, may be used without limitation as long as they can disrupt the cells included in a liquid-phase mixed solution by applying physical impact to the mixed solution.

Because this cell disruptor 120 is known in the art, a detail description thereof will be omitted.

As such, the exosome extraction system 100, 100' or 200 according to one embodiment of the present invention may generate a large amount of a cell lysate through a single process by disrupting a large amount of cells included in the cell fluid through the cell disruptor 120, and may extract a large amount of exosomes e by sequentially filtering the remaining materials other than the exosomes e from the cell lysate in a post-processing step.

The filter unit 130, 130' or 230 may separate the exosomes e from the cell lysate supplied from the cell disruptor 120.

That is, the filter unit 130, 130' or 230 may remove or separate materials other than the exosomes e from the cell lysate. In this way, the filter unit 130, 130' or 230 may convert the cell lysate into a mixed solution including the desired exosomes e, and the cell lysate converted into the mixed solution including the desired exosomes e in the filter unit 130, 130' or 230 may be moved to the recovery unit 140.

At this time, the filter unit 130, 130' or 230 may extract a large amount of high-purity exosomes e by sequentially filtering out each of the materials having different sizes from the cell lysate.

For this purpose, the filter unit 130, 130' or 230 may include a primary filter unit 131, 131' or 231 connected to the rear end of the cell disruptor 120, and a secondary filter unit 132 or 232 connected to the rear end of the primary filter unit 131, 131' or 231, as shown in FIGS. 1, 4, and 5.

In this case, the primary filter unit 131 or 131' may serve to filter or separate proteins having a smaller size than the exosomes e from the cell lysate supplied from the cell disruptor 120, and the secondary filter unit 132 may serve to remove materials having a larger size than the exosomes from the cell lysate that has passed through the primary filter unit 131 or 131'.

For example, the primary filter unit 131 or 131' may separate proteins r, which have a size approximately similar to the size of the exosomes while having the relatively smallest size among the materials included in the cell lysate, from the cell lysate.

As a non-limiting example, the primary filter unit 131 or 131' may remove proteins r having a size of less than 30 nm from the cell lysate, and the secondary filter unit 132 may remove undisrupted cells, cell debris, and microvesicles, which have a size of greater than 200 nm, from the cell lysate.

For this purpose, the primary filter unit 131 or 131' may employ a known tangential flow filtration (TFF) system in which a target material is filtered in a direction perpendicular to the direction in which a solution to be filtered flows, and in which the solution to be filtered is recycled and further purified, as shown in FIG. 2.

In this case, the TFF system may employ a filtration member F1 having pores P1 having a smaller size than the exosomes e while having a larger size than proteins r having a predetermined size, as shown in FIG. 2.

Accordingly, in the process of passing through the primary filter unit 131 or 131', proteins r having a smaller size than the exosomes may be first removed from the cell lysate, and the cell lysate from which the proteins have been removed may be supplied to the secondary filter unit 132.

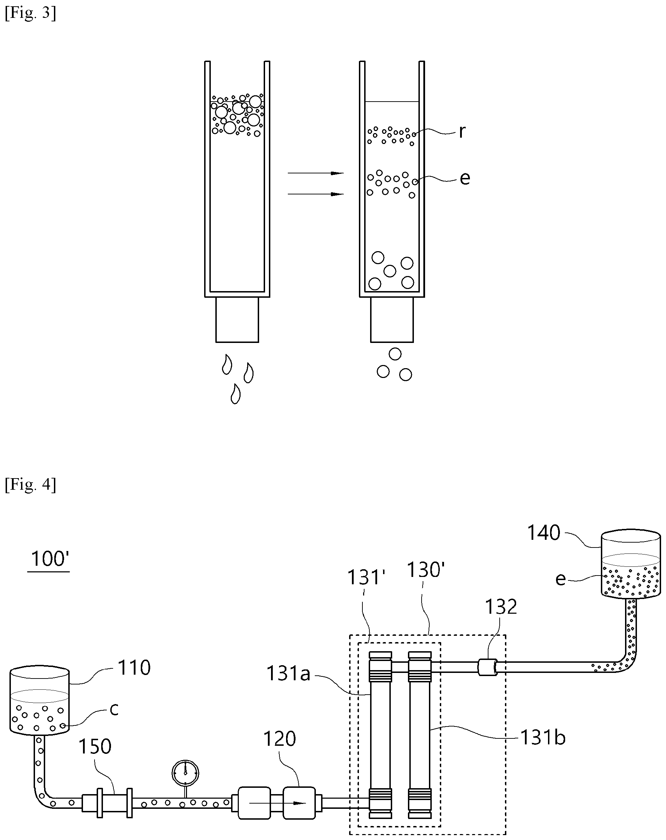

As another example, the primary filter unit 131 or 131' may employ a known size exclusion chromatography system using a porous resin, as shown in FIG. 3.

In this case, the porous resin may include pores having a certain size, and the pores may be formed to have a smaller size than the exosomes while having a larger size than proteins having a certain size.

Accordingly, in the process of passing through the primary filter unit 131 or 131', the proteins r having a smaller size than the exosomes may be first removed from the cell lysate, and the cell lysate from which the proteins have been removed may be supplied to the secondary filter unit 132.

That is, in the process of undergoing size exclusion chromatography, the proteins r having the smallest size among the cell lysate may move more slowly than the undisrupted cells, the cell debris, the microvesicles, and the exosomes, which have a larger size, and move toward the secondary filter unit 132.

In this way, a mixed solution including the undisrupted cells, the cell debris, the microvesicles, and the exosomes, which have a larger size in the cell lysate passing through the primary filter units 131 and 131', may move first to the secondary filter unit 132, and the proteins r, which have the smallest size among the cell lysate, may move to the secondary filter unit 132 after the mixed solution with a time difference.

As a result, the cell lysate that has passed through the primary filter units 131 and 131' may move first to the secondary filter unit 132 in a state in which the proteins r are separated from the cell lysate.

Because such size exclusion chromatography is well known in the art, a detailed description thereof will be omitted.

As described above, the secondary filter unit 132 may be connected to the rear end of the primary filter units 131 and 131', and may filter materials having a larger size than the exosomes from the cell lysate that has passed through the primary filter unit 131 or 131'.

That is, the secondary filter unit 132 may be configured to filter materials having a larger size than the exosomes from the cell lysate from which the proteins r having a smaller size than the exosomes have been removed in the process of passing through the primary filter unit 131 or 131'.

As a non-limiting example, the secondary filter unit 132 may include a filtration member (not shown) having a pore size that allows the exosomes to pass therethrough, and the filtration member may have pores having an average pore size of 200 nm.

Accordingly, as the cell lysate flowing into the secondary filter unit 132 passes through the secondary filter unit 132, undisrupted cells and cell debris having a larger size than the exosomes may be filtered, the cell lysate that has passed through the secondary filter unit 132 may be converted into a mixed solution including the exosomes, and the cell lysate converted to the mixed solution including the exosomes may move toward the recovery unit 140.

However, the pore size of the filtration member employed in the secondary filter unit 132 may be appropriately changed depending on the size of the desired exosomes, but the present invention is not limited thereto. In addition, like the primary filter unit 131 or 131' described above, the secondary filter unit 132 may be composed of a known size exclusion chromatography system using a porous resin. In this case, the porous resin may include pores having a certain size, and the pores may be formed to have a smaller size than the microvesicles while having a larger size than the exosomes.

In this case, the exosome extraction system 100' or 200 according to one embodiment of the present invention may be provided with a plurality of the above-described primary filter units 131.

For example, the exosome extraction system 100' according to one embodiment of the present invention may include a plurality of primary filter units 131a and 131b connected in series with each other between the cell disruptor 120 and the secondary filter unit 132, as shown in FIG. 4.

In this case, each of the plurality of primary filter units 131a and 131b may play the same role. That is, each of the plurality of primary filter units 131a and 131b may serve to filter proteins included in the cell lysate.

As a non-limiting example, each of the plurality of primary filter units 131a and 131b may be provided as the TFF system described above.

As a specific example, the plurality of primary filter units 131a and 131b may include a first TFF system 131a and a second TFF system 131b connected in series between the cell disruptor 120 and the secondary filter unit 132, as shown in FIG. 4.

In this case, the first TFF system 131a and the second TFF system 131b may employ a filtration member F1 having pores having the same size.

In this way, in the exosome extraction system 100' according to this embodiment, the proteins included in the cell lysate may be removed twice while sequentially passing through the two TFF systems 131a and 131b connected in series with each other.

Accordingly, the exosomes that have passed through the secondary filter unit 132 may be converted to a purer state.

However, in this embodiment, each of the plurality of primary filter units 131a and 131b connected in series with each other is not limited to the TFF systems, and each of the plurality of primary filter units 131a and 131b connected in series with each other may be provided with the size exclusion chromatography system described above, or may be composed of a combination of the TFF system and size exclusion chromatography system.

As another example, when the exosome extraction system 200 according to one embodiment of the present invention includes a plurality of primary filter units 231a and 231b connected in series with each other, and each of the plurality of primary filter units 231a and 231b may be configured to play different roles.

That is, any one (231b) of the plurality of primary filter units 231a and 231b may serve to filter the proteins included in the cell lysate, and the other one (231a) of the plurality of primary filter units 231a and 231b may serve to remove the undisrupted cells and cell debris included in the cell lysate.

As a non-limiting example, each of the plurality of primary filter units 231a and 231b may be provided as the TFF system described above.

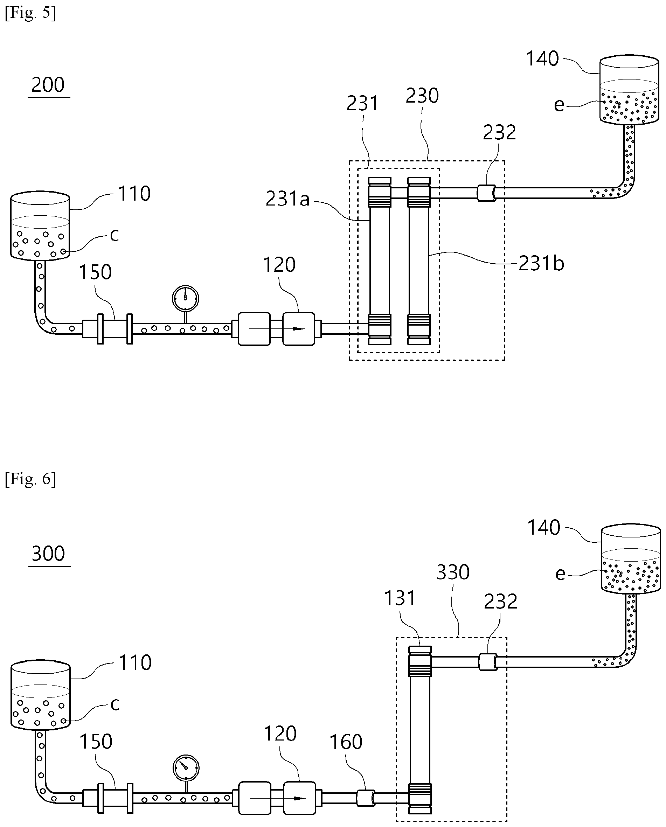

As a specific example, the exosome extraction system 200 according to one embodiment of the present invention may include a plurality of primary filter units 231a and 231b connected in series with each other between the cell disruptor 120 and a secondary filter unit 232, as shown in FIG. 5. In this case, any one of the plurality of primary filter units 231a and 231b may be provided as a first TFF system 231a, and the other one of the plurality of primary filter units 231a and 231b may be provided as a second TFF system 231b.

In this case, the filtration member of the first TFF system 231a and the filtration member of the second TFF system 231b may have different pore sizes.

That is, the first TFF system 231a may employ a filtration member having pores having a larger size than the microvesicles to filter undisrupted cells and cell debris that have a larger size than the microvesicles in the cell lysate, and the second TFF system 231b may employ a filtration member having pores having a smaller size than the exosomes while having a larger size than proteins having a predetermined size.

In this way, the first TFF system 231a may serve as a pre-filter to filter out undisrupted cells and cell debris that have a larger size than the exosomes in the cell lysate, the second TFF system 231b may serve to filter out proteins having a smaller size than the exosomes in the cell lysate, and the secondary filter unit 232 may serve to filter microvesicles that have a smaller size than the undisrupted cells and cell debris while having a larger size than the exosomes in the cell lysate.

That is, in this embodiment, the secondary filter unit 232 may not serve to filter the undisrupted cells, the cell debris, and the microvesicles, which have a larger size than the exosomes, from the cell lysate as in the embodiment described above, but may serve to filter the microvesicles from the cell lysate including the exosomes and microvesicles.

For this purpose, the secondary filter unit 232 may be composed of a filter capable of filtering microvesicles while allowing exosomes to pass therethrough.

Accordingly, in this embodiment, the undisrupted cells and cell debris included in the cell lysate may be filtered through the first TFF system 231a, the proteins included in the cell lysate may be filtered through the second TFF system 231b, and the microvesicles included in the cell lysate may be filtered through the secondary filter unit 232.

In this way, the cell lysate supplied from the cell disruptor 120 may be converted into a mixed solution including exosomes while sequentially passing through the first TFF system 231a, the second TFF system 231b, and the secondary filter unit 232. In this case, the mixed solution including the exosomes, which has passed through the secondary filter unit 232, may be moved to the recovery unit 140.

That is, in this embodiment, the cell lysate supplied to the secondary filter unit 232 may be a mixed solution including microvesicles and exosomes from which undisrupted cells and cell debris having a relatively large size have been removed in the first TFF system 231a, and the secondary filter unit 232 may filter only microvesicles from the mixed solution including the microvesicles and exosomes.

Therefore, in this embodiment, the secondary filter unit 232 does not need to remove undisrupted cells and cell debris having a relatively larger size. Therefore, even when the secondary filter unit 232 employs a filtration member having small-sized pores that allow exosomes to pass therethrough, the small-sized pores may be prevented from being blocked by the undisrupted cells and cell debris.

Accordingly, in this embodiment, the secondary filter unit 232 may smoothly filter out only microvesicles having a larger size than the exosomes.

Meanwhile, an exosome extraction system 300 according to one embodiment of the present invention may further include a pre-filter unit 160 disposed between the cell disruptor 120 and a filter unit 330, as shown in FIG. 6.

For example, the exosome extraction system 300 according to this embodiment may include a raw material supply unit 110, a cell disruptor 120, a pre-filter unit 160, a filter unit 330, and a recovery unit 140, as shown in FIG. 6. In this case, the filter unit 330 may include a primary filter unit 131 and a secondary filter unit 232.

In this case, the raw material supply unit 110 may serve to supply a cell fluid, which is a raw material for extracting exosomes, and the cell disruptor 120 may disrupt the cell fluid supplied from the raw material supply unit 110 into a cell lysate.

Also, the cell lysate converted into the mixed solution including the desired exosomes e while passing through the filter unit 330 may be moved to the recovery unit 140.

In this embodiment, because the raw material supply unit 110, the cell disruptor 120, and the recovery unit 140 are the same as those described above, detailed descriptions thereof will be omitted.

At this time, the pre-filter unit 160 may filter materials having a larger size than the microvesicles, the exosomes, and the proteins among the materials included in the cell lysate.

That is, the pre-filter unit 160 may serve as a pre-filter that filters undisrupted cells and cell debris having a larger size than the microvesicles in the cell lysate.

For this purpose, the pre-filter unit 160 may include a filtration member F2 having pores P2 that have a smaller size than the undisrupted cells and cell debris while having a larger size than the microvesicles.

For example, the filtration member F2 may be a plate-like member having a predetermined area, and the plate-like member may include a membrane having pores having a predetermined size.

As a non-limiting example, the pre-filter unit 160 may be composed of a syringe filter, a bottle filter, a flat membrane filter, and the like known in the art, which includes the filtration member F2.

In this way, in this embodiment, the cell lysate that has passed through the pre-filter unit 160 may be a mixed liquid from which undisrupted cells and cell debris have been removed. In other words, the cell lysate that has passed through the pre-filter unit 160 may be a mixed solution which includes proteins, exosomes, and microvesicles but from which undisrupted cells and cell debris have been removed.

That is, in this embodiment, the pre-filter unit 160 may serve to pre-filter undisrupted cells and cell debris having a larger size than the exosomes and microvesicles. As in the embodiment described above, the primary filter unit 131 may serve to filter proteins having a smaller size than the exosomes from the cell lysate, and the secondary filter unit 232 may serve to filter microvesicles having a larger size than the exosomes from the cell lysate.

Accordingly, in this embodiment, the cell lysate supplied to the secondary filter unit 232 may be a mixed solution including microvesicles and exosomes, from which undisrupted cells and cell debris having a larger size have been removed in the pre-filter unit 160. In this case, the secondary filter unit 232 may filter only microvesicles from the mixed solution including the microvesicles and exosomes.

As a result, in this embodiment, the secondary filter unit 232 does not need to remove undisrupted cells and cell debris having a relatively large size. Therefore, even when the secondary filter unit 232 employs a filtration member having small-sized pores that allow exosomes to pass therethrough, the small-sized pores may be prevented from being blocked by undisrupted cells and cell debris.

Accordingly, in this embodiment, the secondary filter unit 232 may smoothly filter out only microvesicles having a larger size than the exosomes.

In the exosome extraction system 300 according to this embodiment, the primary filter unit 131' shown in FIG. 4 may be employed as the primary filter unit 131, and as in the embodiment described above, a known TFF system, size exclusion chromatography system, and the like may be used as the primary filter unit 131.

In addition, in this embodiment, the secondary filter unit 232 may include a filtration member (not shown) having a pore size that allows the exosomes to pass therethrough, and the filtration member may have pores having an average pore size of 200 nm. In this case, the pore size of the filtration member employed in the secondary filter unit 232 may be appropriately changed depending on the size of the desired exosomes.

Additionally, in this embodiment, the secondary filter unit 232 may be composed of a known size exclusion chromatography system using a porous resin. In this case, the porous resin may include pores having a certain size, and the pores may be formed to have a smaller size than the microvesicles while having a larger size than the exosomes.

Meanwhile, unlike the embodiment described above, an exosome extraction system 400 according to one embodiment of the present invention may be implemented by separating the exosomes and proteins having a relatively smaller size from the cell lysate later.

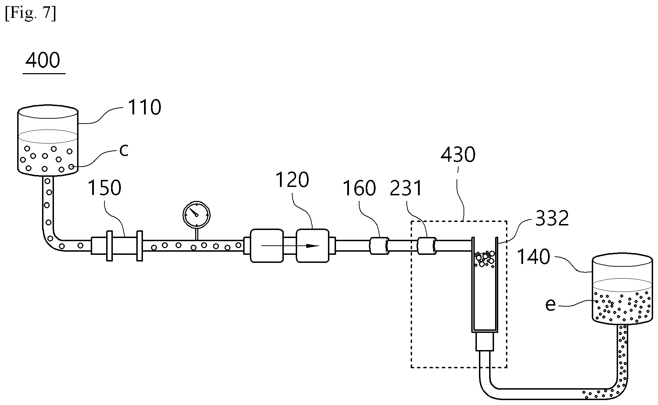

For example, the exosome extraction system 400 according to this embodiment may include a raw material supply unit 110, a cell disruptor 120, a pre-filter unit 160, a filter unit 430, and a recovery unit 140, as shown in FIG. 7.

In this case, the raw material supply unit 110 may serve to supply a cell fluid, which is a raw material for extracting exosomes, and the cell disruptor 120 may disrupt the cell fluid supplied from the raw material supply unit 110 into a cell lysate.

Also, the pre-filter unit 160 may be connected between the cell disruptor 120 and the filter unit 430, and may filter undisrupted cells and cell debris having a larger size than the microvesicles, the exosomes, and the proteins among the materials included in the cell lysate.

In addition, the cell lysate converted into the mixed solution including the desired exosomes e while passing through the filter unit 430 may be moved to the recovery unit 140.

In this embodiment, because the raw material supply unit 110, the cell disruptor 120, the pre-filter unit 160, and the recovery unit 140 are the same as those described above, detailed descriptions thereof will be omitted.

In this case, in this embodiment, the filter unit 430 may include a primary filter unit 231 and a secondary filter unit 332, and the primary filter unit 231 and secondary filter unit 332 may be sequentially connected to the rear end of the pre-filter unit 160, as in the embodiment described above.

In this case, the roles of the primary filter unit 231 and the secondary filter unit 332 of this embodiment may be changed from the roles of the primary filter unit 131 or 131' and the secondary filter unit 132 or 232 of the above-described embodiment.

That is, the primary filter unit 231 may serve to filter microvesicles having a larger size than the exosomes from the cell lysate, and the secondary filter unit 332 may serve to filter proteins having a smaller size than the exosomes from the cell lysate.

For this purpose, the primary filter unit 231 may be connected to the rear end of the pre-filter unit 160, and may filter materials having a larger size than the exosomes from the cell lysate that has passed through the pre-filter unit 160.

That is, the primary filter unit 231 may be configured to filter materials having a larger size than the exosomes from the cell lysate from which undisrupted cells and cell debris, which have a larger size than the exosomes, the proteins, and the microvesicles, have been removed while passing through the pre-filter unit 160.

As a non-limiting example, the primary filter unit 231 may include a filtration member having a pore size that allows the exosomes to pass therethrough, and the filtration member may have pores having an average pore size of 200 nm.

Accordingly, as the cell lysate flowing into the primary filter unit 231 passes through the primary filter unit 231, microvesicles having a larger size than the exosomes may be filtered, and the cell lysate that has passed through the primary filter unit 231 may be converted into a mixed solution including the exosomes and proteins.

In this way, the cell lysate converted into the mixed solution including the exosomes and proteins in the primary filter unit 231 may move toward the secondary filter unit 332.

However, in this embodiment, the pore size of the filtration member employed in the primary filter unit 231 may be appropriately changed depending on the size of the desired exosomes, but the present invention is not limited thereto.

Additionally, in this embodiment, the primary filter unit 231 may be composed of a known size exclusion chromatography system using a porous resin. In this case, the porous resin may include pores having a certain size, and the pores may be formed to have a smaller size than the microvesicles while having a larger size than the exosomes.

The secondary filter unit 332 may serve to filter or separate proteins having a smaller size than the exosomes e from the cell lysate supplied from the primary filter unit 231.

That is, in this embodiment, the secondary filter unit 332 may filter proteins, which have a size approximately similar to the size of the exosomes while having the smallest size among the materials included in the cell lysate, from the cell lysate.

As a non-limiting example, the secondary filter unit 332 may remove proteins having a size of less than 30 nm from the cell lysate.

For this purpose, the secondary filter unit 332 may employ the size exclusion chromatography system described above.

In this case, the porous resin may include pores having a certain size, and the pores may be formed to have a smaller size than the exosomes while having a larger size than proteins having a predetermined size.

Accordingly, in the process of passing through the secondary filter unit 332, the proteins having a smaller size than the exosomes may be first removed from the cell lysate, and the cell lysate from which the proteins have been removed may be supplied to the recovery unit 140.

That is, in the process of passing through the size exclusion chromatography system, proteins having a relatively smaller size in the cell lysate may move more slowly than exosomes having a relatively larger size and move toward the secondary filter unit 332.

In this way, a mixed solution including the exosomes, which have a relatively larger size in the cell lysate passing through the secondary filter unit 332, may move first to the recovery unit 140.

Alternatively, the secondary filter unit 332 may employ the tangential flow filtration (TFF) system described above. In this case, the TFF system may employ a filtration member having pores having a smaller size than the exosomes while having a larger size than proteins having a predetermined size.

Accordingly, in the process of passing through the secondary filter unit 332, proteins having a smaller size than the exosomes may be first removed from the cell lysate, and the cell lysate from which the proteins have been removed may be moved to the recovery unit 140.

Meanwhile, when the above-described pre-filter unit 160 is disposed between the cell disruptor 120 and the filter unit 330 or 430 and the pre-filter unit 160 includes a plate-like filtration member F2 having pores P2 having a predetermined size as described above, an exosome extraction system 500 or 500' according to one embodiment of the present invention may be configured to increase the recovery rate of exosomes included in the cell lysate while extending the lifespan of the filtration member F2.

That is, when the pre-filter unit 160 is disposed between the cell disruptor 120 and the filter unit 330 or 430 as described above, the pre-filter unit 160 may filter undisrupted cells and cell debris having a larger size than the microvesicles, the exosomes, and the proteins among the materials included in the cell lysate.

In other words, because the pre-filter unit 160 filters undisrupted cells and cell debris having the relatively largest size among the materials included in the cell lysate, the undisrupted cells and cell debris may be attached to a surface of the filtration member F2 as the filtration amount of the pre-filter unit 160 increases.

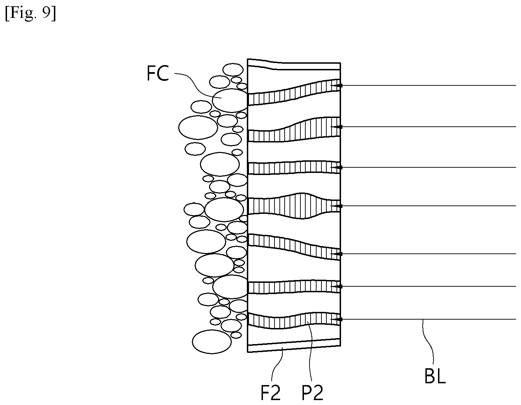

Accordingly, as shown in FIG. 9, a filter cake FK may be generated on a surface of the filtration member F2 due to the undisrupted cells and cell debris, and the filter cake FK generated on the surface of the filtration member F2 may block the pores P2 formed in the filtration member F2.

Because such a filter cake not only reduces the filtration efficiency and lifespan of the filtration member F2, but also blocks the exosomes from moving toward the filter unit 330 or 430 disposed at the rear end of the pre-filter unit 160, the filter cake may reduce a recovery amount of the exosomes recovered by the recovery unit 140.

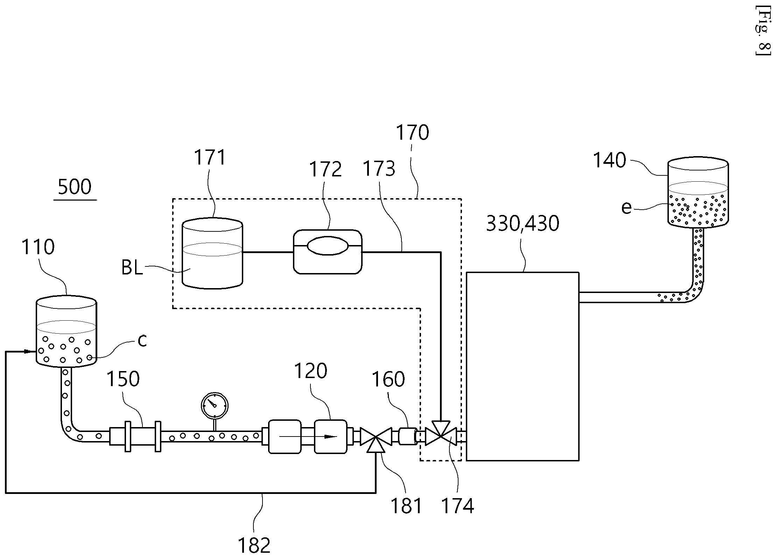

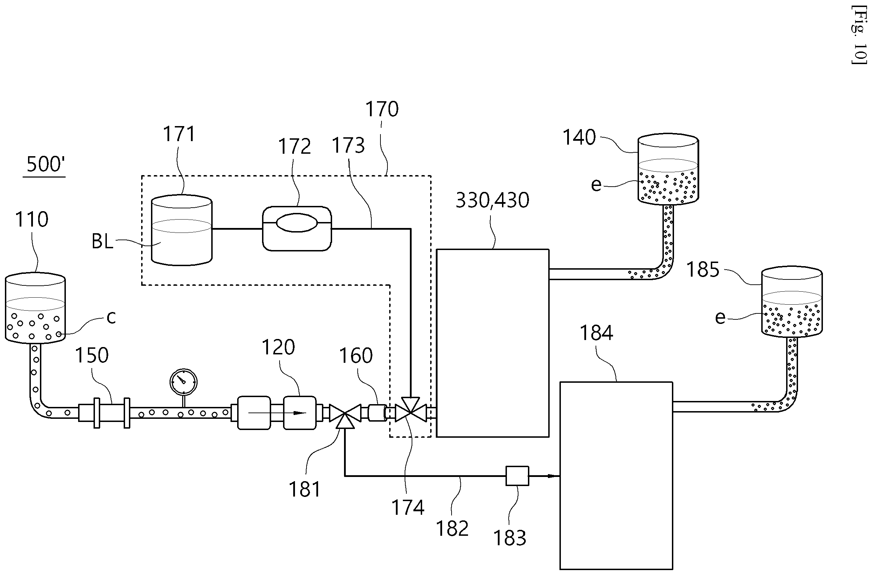

In order to solve this, the exosome extraction system 500 or 500' according to this embodiment may further include a filter cake disruption unit 170 for disrupting and removing the filter cake generated on the surface of the filtration member F2 in the pre-filter unit 160, as shown in FIGS. 8 and 10.

For example, the filter cake disruption unit 170 may include a buffer solution supply unit 171 that stores a predetermined amount of a disruption buffer solution BL, and a supply pump 172 for supplying the disruption buffer solution BL to the pre-filter unit 160 at a predetermined pressure, and the buffer solution supply unit 171 and the supply pump 172 may be connected to the rear end of the pre-filter unit 160 via a supply line 173.

In this case, the filter cake disruption unit 170 may further include a first opening/closing valve 174 installed between the pre-filter unit 160 and the filter unit 330 or 430, and the supply line 173 may have one end connected to the first opening/closing valve 174.

As a non-limiting example, the first opening/closing valve 174 may be a known three-way valve.

As a result, when there is no need to remove the filter cake generated on the surface of the filtration member F2, the first opening/closing valve 174 may be operated to block the cell lysate that has passed through the pre-filter unit 160 from moving to the supply line 173 while allowing the cell lysate to move toward the filter unit 330 or 430.

Accordingly, the cell lysate that has passed through the pre-filter unit 160 may move toward the filter unit 330 or 430, the proteins and microvesicles included in the cell lysate may be removed or separated in the filter unit 330 or 430, and the exosomes included in the cell lysate may pass through the filter unit 330 or 430 and move toward the recovery unit 140.

On the contrary, when it is necessary to remove the filter cake generated on the surface of the filtration member F2, the first opening/closing valve 174 may be operated to block the disruption buffer solution BL stored in the buffer solution supply unit 171 from moving toward the filter unit 330 or 430 while allowing the disruption buffer solution BL to be supplied to the pre-filter unit 160 through the supply pump 172.

At this time, the disruption buffer solution BL may be supplied to the pre-filter unit 160 so that the flow direction of the disruption buffer solution BL can be opposite to the flow direction of the cell lysate flowing from the cell disruptor 120 to the pre-filter unit 160.

Accordingly, the disruption buffer solution BL may be supplied to the pre-filter unit 160 from the buffer solution supply unit 171, the filter cake generated on the surface of the filter member F2 may be disrupted by the disruption buffer solution BL, and the filter cake disrupted by the disruption buffer solution BL may be separated from the surface of the filtration member F2.

In this way, the pores of the filtration member F2 of the pre-filter unit 160 may be secured by disrupting the filter cake, and the filtration member F2 may recover its original function.

That is, the filtration member F2 may block the passage of undisrupted cells and cell debris among the materials included in the cell lysate while allowing proteins, microvesicles, and exosomes among the materials included in cell lysate to pass therethrough.

At this time, the exosome extraction system 500 or 500' according to one embodiment of the present invention does not simply disrupt the filter cake attached to the surface of the filtration member F2 in the pre-filter unit 160 through the above-described filter cake disruption unit 170, but may further extract exosomes from the filter cake disrupted from the surface of the filtration member F2.

For this purpose, the exosome extraction system 500 or 500' according to one embodiment of the present invention may further include a bypass line 182 connected to the front end of the pre-filter unit 160, as shown in FIGS. 8 and 10. In this case, the filter cake disrupted from the surface of the filtration member F2 in the pre-filter unit 160 may move toward to the raw material supply unit 110 as described above or an additional filter unit 184 to be described later through the bypass line 182.

Here, the disrupted filter cake moving toward the raw material supply unit 110 or the additional filter unit 184 through the bypass line 182 may be a mixed solution mixed with a buffer solution and a cell lysate.

In this case, the exosome extraction system 500 or 500' according to one embodiment of the present invention may further include a second opening/closing valve 181 installed between the cell disruptor 120 and the pre-filter unit 160, and one end of the bypass line 182 may be connected to the second opening/closing valve 181.

For example, as shown in FIG. 8, the exosome extraction system 500 according to one embodiment of the present invention may include a second opening/closing valve 181 installed between the cell disruptor 120 and the pre-filter unit 160. In this case, both ends of the bypass line 182 may be connected to the second opening/closing valve 181 and the raw material supply unit 110, respectively.

As a non-limiting example, the second opening/closing valve 181 may be a known three-way valve.

Accordingly, the filter cake disrupted from the surface of the filtration member F2 through the disruption buffer solution BL may move toward the raw material supply unit 110 through the bypass line 182 as the filter cake is blocked from moving toward the cell disruptor 120 through the operation of the second opening/closing valve 181.

As a result, the filter cake FK supplied from the pre-filter unit 160 to the raw material supply unit 110 through the bypass line 182 may be mixed with the cell fluid stored in the raw material supply unit 110, and the filter cake mixed with the cell fluid may be moved to the cell disruptor 120, and then disrupted.

In this way, the filter cake disrupted again in the cell disruptor 120 may be moved to the pre-filter unit 160 and the filter unit 330 or 430 as described above, and the exosomes included in the filter cake may sequentially pass through the cell disruptor 120, the pre-filter unit 160, and the filter unit 330 or 430 and move toward the recovery unit 140.

Accordingly, the exosome extraction system 500 according to this embodiment may further extract exosomes from the filter cake generated in the pre-filter unit 160, thereby increasing the recovery rate of exosomes even when the same amount of cell fluid is used.

In this embodiment, when the filter cake generated on the surface of the filtration member F2 is removed through the filter cake disruption unit 170, the second opening/closing valve 181 may be operated so that the filter cake disrupted from the surface of the filtration member F2 can move toward the raw material supply unit 110 through the bypass line 182.

Also, when there is no need to remove the filter cake generated on the surface of the filtration member F2, the second opening/closing valve 181 may be operated to block the cell lysate moving from the cell disruptor 120 to the pre-filter unit 160 from moving toward the raw material supply unit 110 along the bypass line 182.

As another example, as shown in FIG. 10, the exosome extraction system 500' according to one embodiment of the present invention may further include a second opening/closing valve 181 installed between the cell disruptor 120 and the pre-filter unit 160, and an additional pre-filter unit 183, an additional filter unit 184, and an additional recovery unit 185, which are sequentially installed on the side of the bypass line 182 having one end connected to the second opening/closing valve 181.

That is, in this embodiment, the additional pre-filter unit 183 may be connected in parallel with the pre-filter unit 160 disposed between the cell disruptor 120 and the filter unit 330 or 430, and the additional filter unit 184 and the additional recovery unit 185 may be sequentially connected to the rear end of the additional pre-filter unit 183.

In this case, like the filter unit 330 or 430, the additional filter unit 184 may include a primary filter unit and a secondary filter unit, and the content of the filter unit 330 or 430 described above may be employed in the same manner. In addition, the additional pre-filter unit 183 can also employ the same content as the pre-filter unit 160 described above.

Accordingly, the filter cake disrupted from the surface of the filtration member F2 through the disruption buffer solution BL may move toward the additional pre-filter unit 183 through the bypass line 182 as the filter cake is blocked from moving toward the cell disruptor 120 through the operation of the second opening/closing valve 181.

As a result, the disrupted filter cake bypassed from the pre-filter unit 160 to the additional pre-filter unit 183 through the bypass line 182 may move to the additional filter unit 184 via the additional pre-filter unit 183, and the exosomes included in the filter cake may sequentially pass through the additional pre-filter unit 183 and the additional filter unit 184 and move toward the additional recovery unit 185.

Accordingly, the exosome extraction system 500' according to this embodiment may further extract exosomes from the filter cake generated in the pre-filter unit 160, thereby increasing the recovery rate of exosomes even when the same amount of cell fluid is used.

In addition, when compared to the exosome extraction system 500 according to the embodiment described above, the exosome extraction system 500' according to this embodiment may be configured so that the filter cake disrupted in the pre-filter unit 160 may move toward the additional pre-filter unit 183 connected in parallel with the pre-filter unit 160 through the bypass line 182 without being mixed with the cell fluid in the raw material supply unit 110.

As a result, in the exosome extraction system 500' according to this embodiment, the additional pre-filter unit 183 may increase the filtration efficiency compared to the pre-filter unit 160 of the exosome extraction system 500 according to the embodiment described above.

In this embodiment, when the filter cake generated on the surface of the filtration member F2 is removed through the filter cake disruption unit 170, the second opening/closing valve 181 may be operated so that the filter cake disrupted from the surface of the filtration member F2 can move toward the bypass line 182 through the bypass line 182.

Also, when there is no need to remove the filter cake generated on the surface of the filtration member F2, the second opening/closing valve 181 may be operated to block the cell lysate moving from the cell disruptor 120 to the pre-filter unit 160 from moving toward the raw material supply unit 110 along the bypass line 182 or 192.

Meanwhile, when the pre-filter unit 160 is disposed between the cell disruptor 120 and the filter unit 330 or 430 as described above, an exosome extraction system 600 according to one embodiment of the present invention may be configured to prevent a decrease in the recovery rate of exosomes due to a decrease in the filtration efficiency of the pre-filter unit 160.

That is, when the pre-filter unit 160 disposed between the cell disruptor 120 and the filter unit 330 or 430 filters undisrupted cells and cell debris having a larger size than the microvesicles, the exosomes, and the proteins among the materials included in the cell lysate as described above, a filter cake may be generated on a surface of the filtration member F2 as the filtration amount of the pre-filter unit 160 increases.

Because such a filter cake not only reduces the filtration efficiency and lifespan of the filtration member F2, but also blocks the exosomes from moving toward filter unit 330 or 430 disposed at the rear end of the pre-filter unit 160, the filter cake may reduce a recovery amount of exosomes recovered by the recovery unit 140.

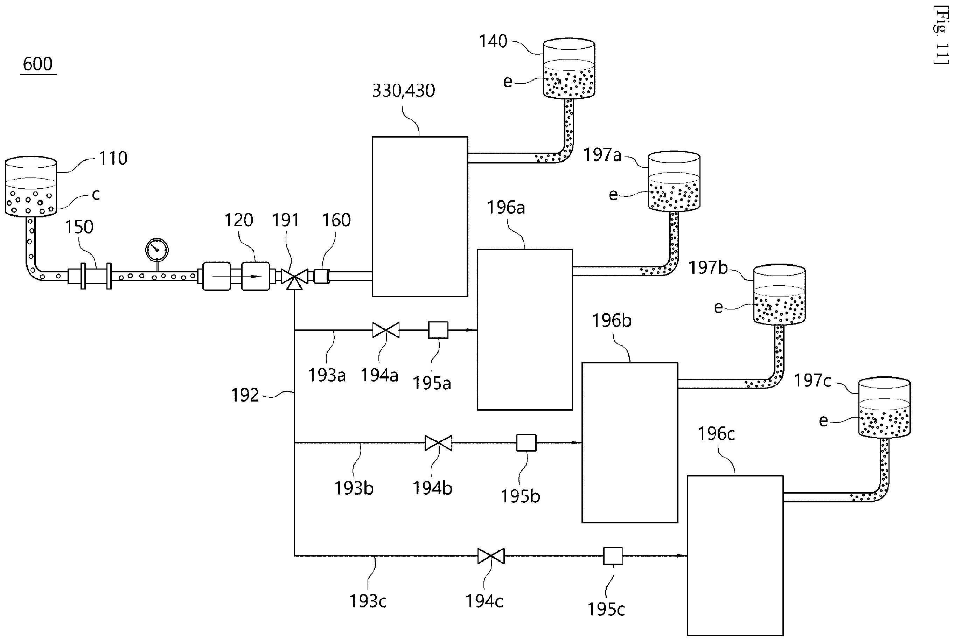

In order to solve this, the exosome extraction system 600 according to this embodiment may further include additional pre-filter units 195a, 195b and 195c connected in parallel with the pre-filter unit 160, as shown in FIG. 11.

That is, the exosome extraction system 600 according to this embodiment may further include additional pre-filter units 195a, 195b and 195c connected in parallel with the pre-filter unit 160 via the bypass line 192, and additional filter units 196a, 196b and 196c and additional recovery units 197a, 197b and 197c connected to rear ends of the additional pre-filter units 195a, 195b and 195c.

In this case, one end of the bypass line 192 may be connected to an opening/closing valve 191 disposed between the cell disruptor 120 and the pre-filter unit 160, and the additional pre-filter units 195a, 195b and 195c, the additional filter units 196a, 196b and 196c, and the additional recovery units 197a, 197b and 197c may be sequentially installed on sides of branch lines 193a, 193b and 193c connected to the bypass line 192.

That is, the bypass line 192 may be connected to the front end of the pre-filter unit 160 via the opening/closing valve 191.

Also, in this embodiment, the additional pre-filter units 195a, 195b and 195c may play the same role as the pre-filter unit 160 of the embodiment described above, the additional filter units 196a, 196b and 196c may play the same role as the filter unit 330 or 430 of the embodiment described above, and the additional recovery units 197a, 197b and 197c may play the same role as the recovery unit 140 of the embodiment described above.

In addition, in this embodiment, the additional pre-filter units 195a, 195b and 195c, the additional filter units 196a, 196b and 196c, and the additional recovery units 197a, 197b and 197c may be configured in the same manner as the pre-filter unit 160, the filter unit 330 or 430, and the recovery unit 140 of the embodiments described above.

As a non-limiting example, three additional pre-filter units 195a, 195b and 195c, three additional filter units 196a, 196b and 196c, and three additional recovery units 197a, 197b and 197c may be provided. In this case, the additional pre-filter units 195a, 195b and 195c, the additional filter units 196a, 196b and 196c, and the additional recovery units 197a, 197b and 197c may be respectively provided on the sides of the three branch lines 193a, 193b and 193c connected to the bypass line 192, and additional opening/closing valves 194a, 194b and 194c for opening/closing a flow path may be installed on the sides of the branch lines 193a, 193b and 193c, respectively.

Accordingly, when the filtration efficiency of the filtration member F2 is reduced due to the filter cake generated on the surface of the filtration member F2 in the pre-filter unit 160, the opening/closing valve 191 may be operated to allow the cell lysate that has passed through the cell disruptor 120 to move toward the bypass line 192, and any one of the three additional opening/closing valves 194a, 194b and 194c may be operated to allow the cell lysate to flow in from the bypass line 192.

Accordingly, the cell lysate that has passed through the cell disruptor 120 does not move toward the pre-filter unit 160, but may selectively move to any one of the three branch lines 193a, 193b and 193c through the bypass line 192.

For example, the cell lysate discharged from the cell disruptor 120 may move toward the first branch line 193a of the three branch lines 193a, 193b and 193c through the operation of the opening/closing valve 191 and the additional opening/closing valves 194a, 194b and 194c. Accordingly, the cell lysate that has moved toward the first branch line 193a may sequentially pass through the additional pre-filter unit 195a and the additional filter unit 196a installed on the first branch line 193a, and the exosomes included in the cell lysate may pass through the additional pre-filter unit 195a and the additional filter unit 196a and move toward the additional recovery unit 197a.

In the same way, when the filtration efficiency of the additional pre-filter unit 195a installed on the first branch line 193a is lowered by the filter cake, the cell lysate may move toward the second branch line 193b of the three branch lines 193 a, 193b and 193c through the operation of the opening/closing valve 191 and the additional opening/closing valves 194a, 194b and 194c. Accordingly, the cell lysate that has moved toward the second branch line 193b may sequentially pass through the additional pre-filter unit 195b and the additional filter unit 196b installed on the second branch line 193b, and the exosomes included in the cell lysate may pass through the additional pre-filter unit 195b and the additional filter unit 196b and move toward the additional recovery unit 197b.

In this way, a process for extracting exosomes may be performed continuously in the exosome extraction system 600 according to one embodiment of the present invention because the cell lysate may move toward other additional pre-filter units 195a, 195b and 195c connected in parallel with the pre-filter unit 160 even when the filtration efficiency of the pre-filter unit 160 is reduced by the filter cake as described above.

Accordingly, the exosome extraction system 600 according to one embodiment of the present invention may increase production yield because it is possible to perform a continuous process.

Meanwhile, in the drawings and the detailed descriptions, a size exclusion chromatography system is exemplified as a chromatography system that may be employed as the primary filter unit 131, 131' or 231 and/or the secondary filter unit 132, 232 or 332, but the present invention is not limited thereto. In this case, various known chromatography systems such as ion exchange chromatography, affinity chromatography, and the like may all be applied.

In addition, it was described that the pre-filter unit 160 also filters undisrupted cells and cell debris included in the cell lysate through a plate-like filtration member having a predetermined area, but the present invention is not limited thereto. In this case, various known filters may be employed as long as they can filter or separate undisrupted cells and cell debris included in the cell lysate.

While one embodiment of the present invention has been described above, the spirit of the present invention is not limited to the embodiments proposed in this specification. Other embodiments may be easily suggested by those skilled in the art by adding, changing and removing components and are included within the spirit and scope of the present invention.

## Claims

1. An exosome extraction system comprising:
a raw material supply unit configured to supply a liquid-phase cell fluid containing cells;
a cell disruptor configured to disrupt the cells through physical impact to convert the cell fluid supplied from the raw material supply unit into a cell lysate including microvesicles, exosomes, and proteins;
a filter unit configured to separate the exosomes from the cell lysate supplied from the cell disruptor; and
a recovery unit configured to recover the exosomes separated from the filter unit.

2. The exosome extraction system of claim 1, wherein the filter unit includes:
a primary filter unit connected to the rear end of the cell disruptor to filter or separate proteins from the cell lysate; and
a secondary filter unit connected to the rear end of the primary filter unit to filter materials having a larger size than the exosomes from the cell lysate that has passed through the primary filter unit.

3. The exosome extraction system of claim 2, wherein the primary filter unit is a tangential flow filtration (TFF) system.

4. The exosome extraction system of claim 2, wherein the primary filter unit is a size exclusion chromatography system.

5. The exosome extraction system of claim 2, wherein the exosome extraction system comprises a plurality of primary filter units connected in series with each other between the cell disruptor and the secondary filter unit, and
the proteins included in the cell lysate are filtered multiple times while sequentially passing through the plurality of primary filter units.

6. The exosome extraction system of claim 1, wherein the filter unit includes:
a primary filter unit connected to the rear end of the cell disruptor; and
a secondary filter unit connected to the rear end of the primary filter unit to filter the microvesicles from the cell lysate that has passed through the primary filter unit,
wherein the primary filter unit includes a plurality of tangential flow filtration (TFF) systems connected in series with each other, and
the plurality of TFF systems include:
a first TFF system configured to filter materials having a larger size than the microvesicles, the exosomes, and the proteins from the cell lysate, and
a second TFF system configured to filter the proteins from the cell lysate that has passed through the first TFF system.

7. The exosome extraction system of claim 1, further comprising a pre-filter unit disposed between the cell disruptor and the filter unit to filter materials having a larger size than the microvesicles, the exosomes, and the proteins from the cell lysate,
wherein the pre-filter unit includes a plate-like filtration member having a predetermined area.

8. The exosome extraction system of claim 7, wherein the filtration member includes a membrane having predetermined pores.

9. The exosome extraction system of claim 7, further comprising a filter cake disruption unit configured to disrupt a filter cake attached to a surface of the filtration member.

10. The exosome extraction system of claim 9, wherein the filter cake disruption unit includes a buffer solution supply unit that stores a certain amount of a disruption buffer solution supplied to the pre-filter unit and a supply pump configured to supply the disruption buffer solution to the pre-filter unit at a predetermined pressure,
the buffer solution supply unit and supply pump are connected to the rear end of the pre-filter unit via a supply line, and
the disruption buffer solution is supplied toward the pre-filter unit so that a flow direction of the disruption buffer solution is opposite to a flow direction of the cell lysate flowing from the cell disruptor to the pre-filter unit.

11. The exosome extraction system of claim 10, wherein the filter cake disrupted from the filtration member through the filter cake disruption unit is supplied to the raw material supply unit via a bypass line.

12. The exosome extraction system of claim 10, further comprising:
an additional filter unit connected to the front end of the pre-filter unit via a bypass line; and
an additional recovery unit connected to the rear end of the additional filter unit to recover the exosomes separated from the additional filter unit,
wherein the filter cake disrupted from the filtration member through the filter cake disruption unit is supplied to the additional filter unit along the bypass line, and
the additional filter unit separates the exosomes from the filter cake disrupted from the filtration member.

13. The exosome extraction system of claim 7, further comprising:
an additional pre-filter unit connected in parallel with the pre-filter unit via a bypass line connected between the cell disruptor and the pre-filter unit, and
an additional filter unit and an additional recovery unit sequentially connected to the rear end of the additional pre-filter unit to correspond one-to-one to the additional pre-filter unit,
wherein the cell lysate generated in the cell disruptor moves to any one of the pre-filter unit and the additional pre-filter unit.

14. The exosome extraction system of claim 7, wherein the filter unit includes:
a primary filter unit connected to the rear end of the pre-filter unit to filter the microvesicles from the cell lysate supplied from the pre-filter unit; and
a secondary filter unit connected to the rear end of the primary filter unit to separate the proteins and the exosomes from the cell lysate that has passed through the primary filter unit.
